# EUROPEAN PATENT SPECIFICATION

(11) **EP 0 672 245 B1**
(45) Date of publication and mention of the grant of the patent: **16.04.1997**
(21) Application number: 93924748.2
(22) Date of filing: 12.11.1993
(51) Int. Cl.: G01N 25/68, G01N 33/00

(54) **HUMIDITY MEASURING INSTRUMENT**
FEUCHTIGKEITSMESSVORRICHTUNG
APPAREIL DE MESURE DE L'HUMIDITE

(30) Priority: 04.12.1992 GB 9225425
(43) Date of publication of application: 20.09.1995
(73) Proprietor: PROTIMETER PLC, Marlow, Buckinghamshire SL7 1LX (GB)
(72) Inventor: DADACHANJI, Fali Minocher, Buckinghamshire SL7 2DE (GB)
(74) Representative: Lesley, Sheila F.
(86) International application number: GB9302325
(87) International publication number: WO9414055

(56) References cited:
- EP-A- 0 264 554
- US-A- 4 240 284
- US-A- 4 478 080

## Description

THIS INVENTION relates to an improved humidity or dewpoint measuring instrument.

It is well known that an accurate and reliable measurement of humidity and/or dewpoint can be made using the well known "chilled mirror" condensation technique. Instruments of this type which operate in a cyclic manner and thus form dew for a few seconds every few minutes probably are the best type. Such instruments can be adapted for dewpoint and humidity measurement. Such instruments are referred to herein as cycling chilled mirror devices.

One of the disadvantages of the above technique is that the time taken between consecutive sampling points is generally from several seconds to several minutes during which time, if the dewpoint or humidity has changed, the onset of this change will only be detected at the next sampling point. For fast monitoring and control purposes this is a disadvantage.

Humidity sensors of other forms are available, for example aluminium oxide, polymer, or conductive film types. These need frequent calibration and are generally not suitable for long term use. However, they respond to changes very quickly and would be ideal as sensing elements for control purposes were it not for their poor long term performance.

US-A-4 240 284 discloses an apparatus employing a cycling chilled mirror type sensor.

It is an object of the present invention to provide apparatus which combines the advantages of the cycling chilled mirror dewpoint measurement technique together with the high speed of response of other humidity sensors, such as aluminium oxide or polymer sensors.

According to this invention there is provided a humidity sensing apparatus comprising a fast response humidity sensor and a cycling chilled mirror type sensor in combination, together with means whereby the calibration of the fast response sensor is periodically checked against the chilled mirror sensor and if necessary corrected.

The checking and correction of calibration may be preferably done by means of analogue correction techniques, for example using analog electronic circuitry, or using digital software techniques.

Embodiments of the invention are described below by way of example with reference to the accompanying drawings, in which:-
FIGURES 1(a), (b) and (c) are schematic diagrams illustrating the operation of an apparatus embodying the invention, incorporating a cycling chilled mirror sensor and a fast response sensor,
FIGURES 2(a) and 2(b) are graphs of waveforms showing the occurrence of short term errors in a humidity monitoring apparatus using only a cycling chilled mirror sensor, and
FIGURES 3(a) to 3(e) show typical waveforms of the chilled mirror sensor, the fast response sensor, and the amalgamated output.

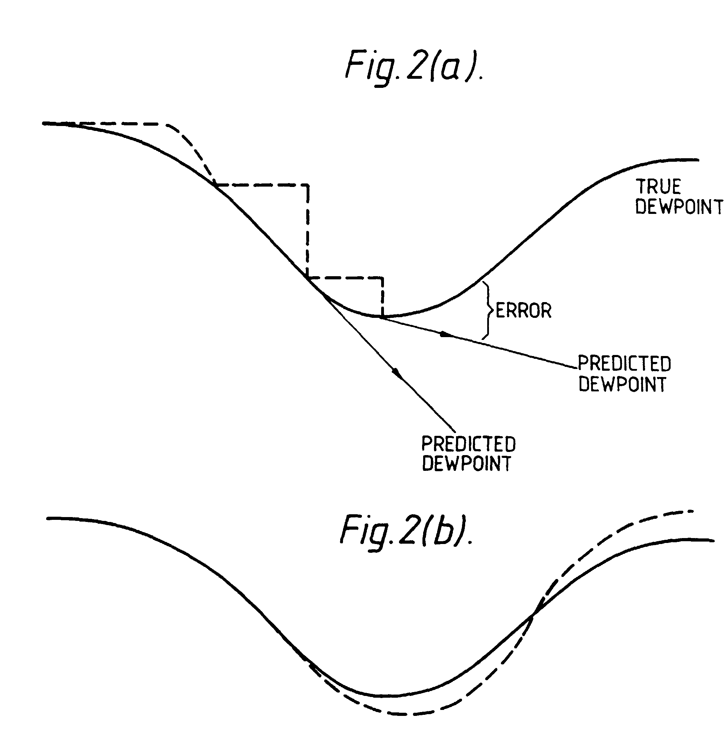

As indicated above, whilst a chilled mirror dewpoint sensing apparatus provides an accurate measure of dewpoint/humidity, at the sample instants at which dew forms on the mirror, even the known cycling chilled mirror types of apparatus, referred to in the introduction, suffer from the disadvantage that the dewpoint/humidity is unknown at times between the sample instants with disadvantages in continuous monitoring applications. Figures 2(a) and 2(b) are graphs in which time is plotted horizontally and temperature (dewpoint) is plotted vertically. Referring to Figure 2(a), the broken line indicates the dewpoint as "perceived" by a monitoring system using such an instrument in a situation where the real dewpoint is varying as indicated in the solid graph.

To reduce the disparities between the "perceived" and real dewpoint illustrated in Figure 2(a), the measurements from the cycling chilled mirror dewpoint sensor may be processed by data processing means such as a microcomputer. Software can be written for such processing means which looks at the periodic measurements made by the cycling chilled mirror dewpoint sensor and which attempts to predict "intelligently" the magnitude and direction of the variation in dewpoint for the intervals between sampling points. Figure 2(b) represents the results which may be achieved in this way, with the predicted variation in the regions between sample instants being shown in broken lines and the true dewpoint variations over the same period being again shown in solid lines. The disadvantage here is that at any one time when the dewpoint/humidity changes direction so as to be altering in the opposite sense from the predicted variation, wrong information could be sent to a control system receiving the output from the processing means.

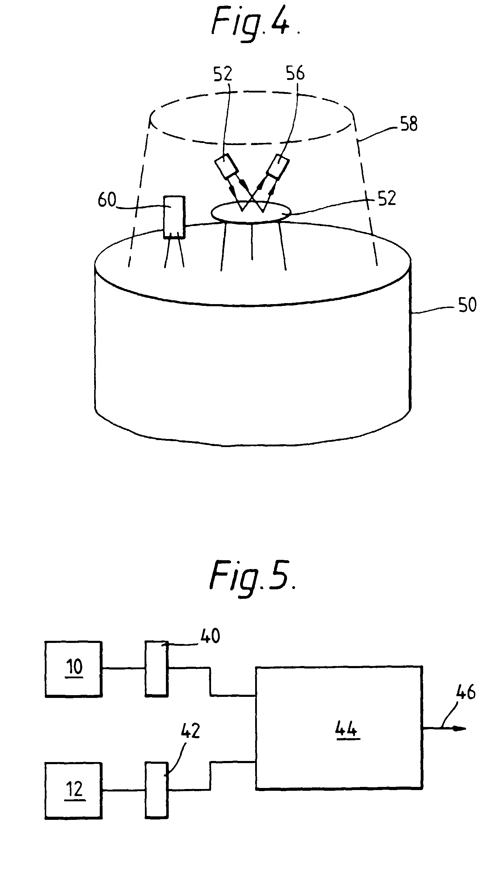

In an embodiment of the invention, the output of a fast response humidity sensor is monitored at the same time as the samples from the chilled mirror device and used to indicate trends in dewpoint/humidity, allowing the last-noted disadvantage to be avoided or minimised. As shown schematically in the block diagram of Figure 5, the fast response sensor 10 and CCM sensor 12 are linked, via respective interfaces 40, 42, to a microprocessor 44, which provides on an output 46, in digital or analog form, a corrected dewpoint signal.

In this embodiment, appropriate software is used in the microprocessor whereby the output of the fast response sensor 10 is used to determine the direction of humidity change between dewpoint sampling points, but the actual values relative to which these changes occur are taken as the values determined by the chilled mirror sensor 12 at the sampling points.

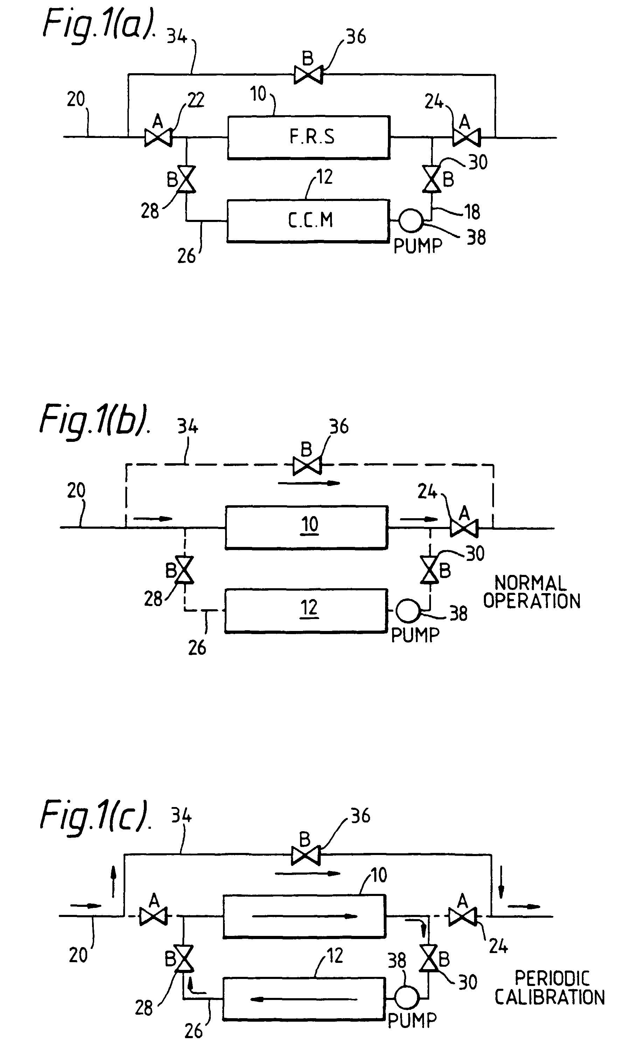

Referring to Figures 1(a) to 1(c), these show an arrangement in which a fast response sensor (FRS) 10 is mounted in a pipe or conduit 20 for normal continuous monitoring of dewpoint or humidity. A solenoid valve 22 is provided in the pipe 20 upstream of the sensor 10 and a further solenoid valve 24 is provided in the pipe 20 downstream of the sensor 10. The calibration of sensor 10, if left unattended, could be erroneous over a period of time. However, as shown in Figure 1(a), the apparatus comprises a cycling chilled mirror humidity/dewpoint measuring device (CCM) 12, referred to herein as the CCM sensor and which is, in effect, a complete conventional chilled-mirror dewpoint/humidity sensing instrument, including electronic circuitry controlling the periodic cooling of the mirror, sensing of dew formation, temperature, etc. The CCM device 12 is connected in a shunt conduit 26 which has its upstream end connected with pipe 20 at a location between solenoid valve 22 and sensor 10 and which has its downstream end connected with pipe 20 at a location between sensor 10 and solenoid valve 24. A solenoid valve 28 is provided in shunt conduit 26 upstream of CCM device 12 and a solenoid valve 30 is provided in shunt conduit 26 downstream of CCM device 12. A pump 38 is provided in shunt conduit 26. In the arrangement shown, the pump 38 is connected between CCM device 12 and valve 30. A shunt conduit 34, which is normally closed by a solenoid valve 36, allows the apparatus as a whole to be by-passed. The fast response humidity sensor 10 may be, for example, of the aluminium oxide, polymer or conductive film type. The valves 28 and 30 are normally closed whilst the valves 22 and 24 are normally open.

In normal operation, a gas of which the moisture content is to be monitored is passed along pipe or conduit 20 and passes *via* valve 22, to fast response sensor 10 and thence, *via* valve 24, as indicated by the flow arrows in Figure 1B.

Periodically, the valve 36 is opened, valves 22 and 24 closed and valves 28 and 30 opened and the pump 38 activated. Thus, the normal gas flow through conduit 20 is diverted through the by-pass 34 whilst the fast response sensor 10 and the CCM sensor 12 are connected, in a closed loop, with pump 38, *via* valves 28 and 30 but are isolated from the flow line 20. By operation of the pump 38, the fluid within this closed loop is continuously circulated, thereby ensuring that the fast response sensor 10 and the CCM sensor 12 are subjected to the same humidity. This allows the output, for the time being, of the fast response sensor 10 at a precisely determined humidity (determined by CCM sensor 12) to be accurately determined, for checking the calibration of the fast response sensor. At the end of this, the calibration phase, the pump 38 is stopped, the valves 28, 30 and 36 closed and the valves 22 and 24 again opened to return the apparatus to its normal mode of operation. The duration of the calibration phase in each cycle, is short in relation to the time for which the apparatus is in its normal condition. If the calibration of the fast response sensor 10 is found, during the calibration phase, to have drifted, a correction in software can be automatically made before the system reverts to its normal working mode. It will be appreciated that the solenoid valves 22, 24, 28, 30 and 36 are preferably controlled by the processor 44 in order to operate in the appropriate sequence and phase at the appropriate intervals.

A variant of this arrangement could be particularly beneficial where, for instance, if the normal measuring mode called for an intrinsically safe sensor/instrument, the fast response sensor with intrinsically safe characteristics could be fitted for operation in a hazardous gas. However, for periodic automatic calibration, a different gas, for example air, could be used in the closed loop to set up the correct calibration. It is not even necessary to calibrate it exactly at the dewpoint being measured as most devices have a very predictable characteristic over a wide range of dewpoints/humidities.

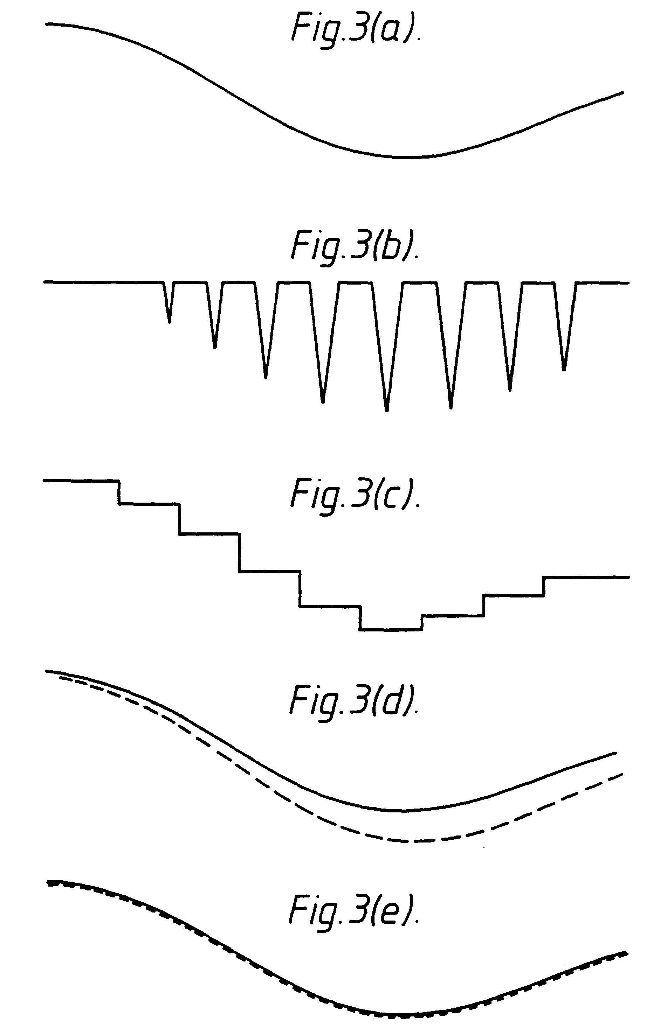

Figures 3(a) to 3(e) are graphs of temperature (dewpoint) (plotted vertically) against time (plotted horizontally). Figures 3(a) to 3(e) will be largely self-explanatory, with the assistance of the following notes. The graph of Figure 3(a) shows a variation of real dewpoint with respect to time, corresponding to variation in moisture content in a gas passed through conduit 20; Figure 3(b) shows the corresponding mirror temperature waveform in a conventional cycling chilled mirror dewpoint measuring apparatus, the minimum temperature values in the troughs indicating the dewpoint at the respective time; Figure 3(c) shows the unprocessed output of such a CCM apparatus; Figure 3(d) shows the corresponding possible output of an uncorrected fast response sensor (in broken lines) with the real dewpoint being reproduced in a solid line for comparison, and Figure 3(e) indicates, for the same humidity variations, the corrected dewpoint signal from the microprocessor 44 and illustrates the close tracking between real dewpoint (solid line) and corrected fast response sensor (broken line) possible with apparatus embodying the invention. As illustrated at 3(c) the output from the CCM sensor, although accurate at the sensing instants, is necessarily step-like in character. The output of the fast response sensor is substantially continuous but may, as illustrated in Figure 3(d), suffer from drift. The apparatus of the invention, as illustrated in Figure 3(e) allows substantially continuous monitoring, with minimal drift.

It will be appreciated that the invention is in no way confined to the metering of the humidity of a gas passing along a pipeline. Indeed, it is envisaged that the widest use of the invention may be in the accurate continuous monitoring of ambient humidity in various locations. Figure 4 illustrates a sensor head of an instrument embodying the invention, which may be used for monitoring ambient humidity. In this instrument, the chilled mirror 52 of a cycling chilled mirror dewpoint sensor is mounted at the end of a probe or other support 50, as is the associated optical bridge (comprising light emitter 54 and photosensor 56) forming part of the CCM sensor, in a manner known *per se* in the art. A fast response humidity sensor (FRS) 60 is also mounted at the end of the probe or support 50, in close proximity to the chilled mirror 52. The fast response sensor, chilled mirror and optical bridge are enclosed in a permeable housing 58, which acts as a dust filter and protects the sensors against mechanical damage. The CCM sensor and the fast response sensor are operated in the same general manner as described with reference to Figures 1(a) to 1(c) and 5, except, of course, that both the fast response sensor and the chilled mirror are continuously exposed to the atmosphere being monitored. As before, each dewpoint determination of the CCM sensor is used to check, and if necessary adjust, the calibration of the fast response sensor.

The invention allows various forms of humidity measuring instruments to be provided having the accuracy and performance of high quality reference instrumentation having a speed of response which is very much faster.

It will be appreciated that whereas, in the above, the periodic recalibration of the fast response sensor has been described as being effected by data processing means, this function may instead, if desired, be performed by means of analogue correction techniques, for example using purpose-built analog computing circuitry, to serve a corresponding function.

## Claims

1. A humidity sensing apparatus comprising a fast response humidity sensor (10) and a cycling chilled mirror type sensor (12) in combination with means for periodically checking the calibration of the fast response sensor against the chilled mirror sensor and correcting if necessary.

2. A humidity sensing apparatus according to claim 1 wherein said means whereby the calibration is periodically checked comprises data processing means (44) arranged to receive sensing information from said fast response sensor (10) and said chilled mirror sensor (12) and to provide at an output a signal (46) significant of the humidity sensed by said fast response sensor and based upon the output from said fast response sensor and the most recent re-calibration established for said fast response sensor, the data processing means being arranged, at intervals, to re-establish a calibration for said fast response sensor on the basis of a dewpoint determination made by said chilled mirror type sensor.

3. A humidity sensing apparatus according to claim 1 or claim 2 including a pipeline (20) for flow of a gas of which the moisture content is to be determined, said fast response sensor (10) being connected in said pipeline, first valve means (22) in said pipeline upstream of said fast response sensor, second valve means (24) in said pipeline downstream of said fast response sensor, a shunt conduit connected across said fast response sensor and extending from a junction with said pipeline at a location between said first valve means and said fast response sensor to a junction with said pipeline at a location between said fast response sensor and said second valve means, a chilled mirror sensor (12) and a pump (38) in said shunt conduit, third valve means (28) in said shunt conduit between said chilled mirror sensor and said pipeline, on the upstream side of said chilled mirror sensor, fourth valve means (30) in said shunt conduit between said chilled mirror sensor and said pipeline, on the downstream side of said chilled mirror sensor, whereby, in normal operation, with said first and second valve means open and said third and fourth valve means closed the gas to be monitored can pass continuously through said fast response sensor and whereby, for calibration of the fast response sensor, said first and second valve means can be closed, said third and fourth valve means opened and said pump operated to circulate gas around a closed circuit including said fast response sensor and said chilled mirror sensor, for calibration of said fast response sensor by reference to the chilled mirror sensor.

4. A humidity sensing apparatus according to claim 1 or claim 2 wherein said fast response sensor (10) and said chilled mirror sensor (12) are mounted close to one another on support means in a common space to share an ambient atmosphere the humidity of which is to be monitored.

## Patentansprüche

1. Feuchtigkeitsmeßvorrichtung, umfassend einen schnellansprechenden Feuchtigkeitssensor (10) und einen Sensor (12) eines zirkulierenden, gekühlten Spiegeltyps in Verbindung mit einem Mittel zum periodischen Überprüfen der Kalibrierung des schnellansprechenden Sensors gegen den gekühlten Spiegelsensor und zum Korrigieren, falls notwendig.

2. Feuchtigkeitsmeßvorrichtung nach Anspruch 1, dadurch gekennzeichnet, daß besagtes Mittel, durch das die Kalibrierung periodisch überprüft wird, ein Datenbearbeitungsmittel (44) umfaßt, das angeordnet ist, um Meßinformation von besagtem schnellansprechenden Sensor (10) sowie von besagtem gekühlten Spiegelsensor (12) zu empfangen und an einem Ausgang ein Signal (46) bereitzustellen, das für die von besagtem schnellansprechenden Sensor gemessene Feuchtigkeit signifikant ist und auf dem Ausgabesignal von besagtem schnellansprechenden Sensor sowie der letzten Re-Kalibrierung, durchgeführt für besagten schnellansprechenden Sensor, basiert, wobei das Datenbearbeitungsmittel angeordnet ist, um, in Intervallen, eine Kalibrierung für besagten schnellansprechenden Sensor auf der Grundlage einer Taupunktsbestimmung, gemacht von besagtem Sensor des gekühlten Spiegeltyps, zu re-etablieren.

3. Feuchtigkeitsmeßvorrichtung nach Anspruch 1 oder 2, gekennzeichnet durch eine Rohrleitung (20) für den Fluß eines Gases, dessen Feuchtigkeitsgehalt zu bestimmen ist, wobei besagter schnellansprechende Sensor (10) in besagter Rohrleitung angeschlossen ist, ein erstes Ventilmittel (22) in besagter Rohrleitung, stromaufwärts besagten schnellansprechenden Sensors, ein zweites Ventilmittel (249 in besagter Rohrleitung, stromabwärts besagten schnellansprechenden Sensors, eine Nebenleitung, die über besagten schnellansprechenden Sensor angeschlossen ist und sich von einer Verbindung mit besagter Rohrleitung an einer Stelle zwischen besagtem ersten Ventilmittel und besagtem schnellansprechenden Sensor zu einer Verbindung mit besagter Rohrleitung an einer Stelle zwischen besagtem schnellansprechenden Sensor und besagtem zweiten Ventilmittel erstreckt, einen gekühlen Spiegelsensor (12) und eine Pumpe (38) in besagter Nebenleitung, ein drittes Ventilmittel (28) in besagter Nebenleitung zwischen besagtem gekühlten Spiegelsensor und besagter Rohrleitung, stromaufwärts besagten gekühlten Spiegelsensors, ein viertes Ventilmittel (30) in besagter Nebenleitung zwischen besagtem gekühlten Spiegelsensor und besagter Rohrleitung, stromabwärts besagten gekühlten Spiegelsensors, wodurch, im Normalbetrieb, in dem besagtes erstes und zweites Ventilmittel geöffnet und besagtes drittes und viertes Ventilmittel geschlossen sind, das zu überwachende Gas kontinuierlich durch besagten schnellansprechenden Sensor hindurchtreten kann, und wodurch, zur Kalibrierung des schnellansprechenden Sensors, besagtes erstes und zweites Ventilmittel geschlossen sein können, besagtes drittes und viertes Ventilmittel geöffnet sein können und besagte Pumpe arbeiten kann, um Gas entlang eines geschlossenen Kreises, enthaltend besagten schnell ansprechenden Sensor und besagten gekühlten Spiegelsensor, zirkuliert, zur Kalibrierung besagten schnellansprechenden Sensors durch Bezugnahme auf den gekühlten Spiegelsensor.

4. Feuchtigkeitsmeßvorrichtung nach Anspruch 1 oder 2, dadurch gekennzeichnet, daß besagter schnellansprechende Sensor (10) und besagter gekühlte Spiegelsensor (12) nahe aneinander auf einem Stützglied in einem gemeinsamen Raum angebracht sind, um sich eine Umgebungsatmosphäre der zu überwachenden Feuchtigkeit zu teilen.

## Revendications

1. Appareil de mesure de l'humidité comprenant un capteur d'humidité à réponse rapide (10) et un capteur de type à miroir refroidi d'itération (12) en combinaison avec des moyens pour vérifier périodiquement l'étalonnage du capteur à réponse rapide par rapport au capteur à miroir refroidi et pour le corriger au besoin.

2. Appareil de mesure de l'humidité selon la revendication 1, dans lequel les moyens permettant de vérifier périodiquement l'étalonnage comprennent des moyens de traitement de données (44) aptes à recevoir des informations de mesure en provenance du détecteur à réponse rapide (10) et du détecteur à miroir refroidi (12) pour fournir en sortie un signal significatif de l'humidité captée par le détecteur à réponse rapide et basé sur la sortie en provenance du détecteur à réponse rapide et du réétalonnage le plus récent établi pour le capteur à réponse rapide, les moyens de traitement de données étant aptes, à certains intervalles, de réétablir un étalonnage pour le capteur à réponse rapide sur la base d'une détermination du point de rosée effectuée par le capteur de type à miroir refroidi.

3. Appareil de mesure d'humidité selon la revendication 1 ou la revendication 2, comprenant une canalisation (20) pour l'écoulement d'un gaz dont il s'agit de déterminer la teneur en humidité, ce capteur à réponse rapide (10) étant connecté à la canalisation, des premiers moyens de vanne (22) dans la canalisation en amont du capteur à réponse rapide, des seconds moyens de vanne (24) dans la canalisation en aval du capteur à réponse rapide, une conduite de dérivation connectée sur le capteur à réponse rapide et allant d'un embranchement avec la canalisation en un emplacement situé entre la première vanne et le capteur à réponse rapide jusqu'à un embranchement avec la canalisation en un emplacement entre le capteur à réponse rapide et la seconde vanne, un détecteur à miroir refroidi (12) et une pompe (38) dans la conduite de dérivation, des troisièmes moyens de vanne (28) dans la conduite de dérivation entre le détecteur à miroir refroidi et la canalisation, sur le côté amont du capteur à miroir refroidi, des quatrièmes moyens de vanne (30) dans la conduite de dérivation entre le capteur de miroir refroidi et la canalisation, sur le côté aval du détecteur à miroir refroidi, permettant dans le fonctionnement normal, avec les premiers et seconds moyens de vanne ouverts et les troisièmes et quatrièmes de vanne fermés de contrôler que le gaz passe en continu entre le premier capteur à réponse rapide et ainsi, pour l'étalonnage du premier capteur à réponse rapide, les premiers et seconds moyens de vanne pourront être fermés, les troisièmes et quatrièmes moyens de vanne pourront être ouverts et la pompe pourra être mise en route pour faire circuler le gaz dans un circuit fermé incorporant le capteur à réponse rapide et le capteur à miroir refroidi, pour l'étalonnage du capteur à réponse rapide en référence au capteur à miroir refroidi.

4. Appareil de détection d'humidité selon la revendication 1 ou la revendication 2, dans lequel le capteur à réponse rapide (10) et le capteur à miroir refroidi (12) sont montés à proximité l'un de l'autre sur des moyens de support dans un espace commun partageant l'atmosphère ambiant dont il s'agit de contrôler l'humidité.
